# EUROPEAN PATENT SPECIFICATION

(11) **EP 0 615 128 B1**
(45) Date of publication and mention of the grant of the patent: **10.09.2003**
(21) Application number: 93203717.9
(22) Date of filing: 29.12.1993
(51) Int. Cl.: G01N 33/543, G01N 33/569

(54) **Equipment and test for determining an antigen/antibody in a test sample**
Vorrichtung und Test zur Bestimmung eines Antigens oder eines Antikörpers in einer Testprobe
Equipement et test pour déterminer un antigène ou un anticorps dans un échantillon

(30) Priority: 12.03.1993 IT MI930477
(43) Date of publication of application: 14.09.1994
(73) Proprietor: LOFARMA S.p.A., 20143 Milano (IT)
(72) Inventor: Falagiani, Paolo, I-20131 Milano (IT); Mistrello, Giovanni, I-20135 Milano (IT)
(74) Representative: Siniscalco, Fabio

(56) References cited:
- EP-A- 0 250 137
- EP-A- 0 296 398
- EP-A- 0 498 124
- WO-A-89/03044
- WO-A-92/01051
- WO-A-93/12426

## Description

The present invention relates to a method of testing for determining an antigen or antibody in a test sample and to equipment for carrying out the test.

More particularly the invention relates to a sandwich type method in which the test sample suspected of containing particular antigens or antibodies is placed in contact with a solid substrate having an antibody or antigen bound to its surface which can form an immunocomplex with the antigen or antibody in the test sample and in which the immunocomplex which may have formed is detected with the use of a marked antibody/antigen as a detector.

EP-A-0 296 398 discloses a one-step immunoassay method to detect the presence of antibodies (or antigens), wherein the antigens are blotted onto a solid substrate, and detecting reagent and a serum as a source of antibodies are added, and all three of these components incubated together.

EP-A-0 498 124 discloses, in particular, an immunoassay for detecting DERF II, which is a major allergen of a house dust mite, wherein a sample, in the form of an extract, is incubated with a monoclonal antibody selectively recognizing DERF II.

WO-A-92 01051 discloses a process of diagnosis in vitro of human or animal hydatidose comprising putting into contact biological material with immunogen peptide sequences directed against monoclonal antibodies.

The invention provides, in particular, a rapid testing method which can be carried out easily even by non-expert operators and enables the response to be displayed directly while at the same time having a high degree of sensitivity.

To this end, the subject of the invention is a method of testing of the type specified above, characterised in that the solid substrate is placed in contact simultaneously with the test sample and with the marked antibody or antigen.

The term "simultaneously" as used in the paragraph above is intended to indicate the following procedures:
- premixing of the test sample and the marked antibody or antigen and contact of the premixed product with the substrate;
- addition of the test sample to the substrate and immediate addition of the marked antibody or antigen without intermediate washing stages.

In the preferred embodiment, the detector antibody or antigen is constituted by an antibody or antigen conjugated with a colloidal dye.

Although the method of the invention is of general application, in view of the objects it is intended to achieve, it is particularly suitable and advantageous for the determination of environmental allergens and for the determination of antibodies resulting from infectious and parasitic diseases.

In one embodiment, the test of the invention is particularly concerned with the determination of the presence of allergens in the domestic environment.

The first indication of the possible role of mites in causing allergies arose in about the 1920s; more recently, around the 1960s, a further series of experiments showed unequivocally that mites of the genus Dermatophagoides (pteronyssinus and farinae), and particularly their excrements, contain a considerable quantity of an allergen termed Der p I, and constitute the most important cause of allergy in house dust.

Since then confirmations have been ever more numerous, to such an extent that the conviction has arisen that allergy to mites in house dust is a worldwide problem, causing a series of clinical manifestations ranging from asthma, to rhino-conjunctivitis to atopic dermatitis, as much in adults as in children. It is thought that from 5 to 10% of the European population is affected by forms of allergy caused by the presence of mites and a further percentage, although not yet sensitized, could become so as a result of prolonged exposure to mites and their faeces. Recent epidemiological studies have shown that the persistence of levels of the allergen Der p I of from 0.5-2µg/g of dust, equivalent to from 25-100 mites/g of dust, is accompanied by an increased risk of allergy arising. For this reason, the control of the environmental concentrations of mites falls within the field of preventive medicine.

The "distribution" of mites may vary according to habitational environments and is an important factor in the modulation of respiratory symptoms. The identification of "places" infested with mites within a home is thus extremely important since it enables procedures for decontaminating the environment to be put into practice (for example the use of acaricides), enabling the elimination of colonies of mites and the removal of faeces which are an important source of allergens. These preventive measures associated with a suitable immunotherapeutic treatment with desensitising extracts for the patient are known to give a clinical improvement. A particular contribution to providing an effective control for mites in house dust is the development of methods of evaluation other than microscopic evaluation, which are rapid, simple and sufficiently sensitive.

The microscopic technique, although having enabled the presence of mites in house dust to be shown for the first time, and having thus demonstrated their potential *role* as a cause of allergy, is certainly not the best method for routine analysis. It remains an indispensable instrument for the researcher who wishes to show the predominant species in the varies geographical regions or who wishes to study the biological characteristics of the various mites but, when applied to routine analysis of dust samples, it has a series of disadvantages:
- the preparation of the sample to be examined and the microscopic evaluation itself are very laborious, time consuming operations and require suitably trained personnel;
- it does not identify the faeces which contain considerable quantities of allergen.

In recent years new techniques have been made available for identifying allergens present in the domestic environment. One of these is based on the use of monoclonal antibodies specific to the allergen (Der p I) of mite faeces in house dust.

The method consists of fixing a suitable quantity of anti-Der p I monoclonal antibody in the well of a microtitre plate followed by incubation with the dust sample and with a second antibody (monoclonal or polyclonal), specific to DerpI, conjugated with biotin. The reaction is displayed colorimetrically with the use of the enzyme peroxidase conjugated with streptavidin and then a suitable substrate.

The method is very sensitive and specific but, like observation with the microscope, requires trained personnel and special laboratory equipment (reader for the colorimetric reaction).

Despite the complexity of this procedure, the method is suitable for tests even of a routine type.

This method has a series of limitations: the length of the test (not less than 3 hours), the multitude of steps in the method, recognition of a single allergen, even though this is important.

A technological and conceptual leap has enabled a kit to be developed which is based on the identification of a marker, guanine, which is the final catabolite of the nitrogen catabolism of arachnidae in general. Guanine is thus present in mite faeces as well since mites belong to the same family. The finding of guanine in house dust could thus show the presence of mites in the environment.

The method is of a semi-quantitative type, easy to use, quick and applicable in routine tests. The results are expressed on the basis of a test carried out on a solid substrate (stick) with the development of a colorimetric reaction, the intensity of which is shown on a chromatic scale.

Given its characteristics of simplicity, it has the advantage that it can be carried out by non-expert personnel.

The disadvantages are:
- lack of specificity, particularly in cases of low positive results. The method is based on the assumption that most of the guanine detectable in house dust derives solely from mite faeces which is not always true. For example the presence of spiders may interfere with the test;
- the allergen in mite faeces is only one of the numerous allergens present in mites. An important allergen (termed Der p II) is, on the contrary, absent from the faeces as it is derived from the mite body; the test does not enable this to be identified.

The lack of specificity and lack of recognition of other important mite allergens are the two negative aspects of this test which cause some perplexity in those carrying out the work.

The method developed by the applicant and described below, when applied particularly to the determination of mite allergens in house dust, enables the disadvantages of the known tests to be overcome while retaining similar characteristics in terms of simplicity of use, direct display of the response (no dependence on specific reading apparatus), sufficient rapidity of realisation.

The method is carried out with the use of antibodies, preferably polyclonal antibodies (obtained for example from rabbits) in a "sandwich" and is able to recognise all the various allergens present in mites and not just Der p I. Experiments with sodium dodecylsulphate gel - electrophoresis on polyacrylamide gel (SDS - PAGE) with transfer of the allergens separated onto nitrocellulose (blotting) paper and subsequent contact with mite antiserum have clearly shown this fact.

The polyclonal antibody specific to mites is placed on a solid substrate, preferably nitrocellulose, constituting the so-called "reactive strip". Subsequently this reactive strip is placed in a test tube to which there are then added simultaneously the dust sample to be examined and the detector antibody (anti-mite antibody conjugated with a colloidal dye).

The development of a coloured spot indicates the presence of mites in the house dust.

It has been found that the combination into a single step of the steps of addition of the sample and addition of the detector reagent conventionally kept separate and spaced by washings, as well as contributing a greater simplicity and rapidity of execution, unexpectedly leads to an improvement in the sensitivity of the method, giving a more marked signal. The same procedure, when carried out with the step of addition of the sample separated from that of the addition of the detector is significantly less suited to use in the analysis of samples of house dust, as shown by the lack of recognition of several positive results, which are, on the contrary, detected by the "one step" version of the method carried out in accordance with the invention, and by examination under the microscope. One possible theory for explaining this phenomenon is that the "one step" version induces the formation of an immunocomplex, (mite) antigen/antibody, which, in precipitating, would facilitate capture of the mite allergens by the mite antibodies fixed to the nitrocellulose paper.

By interposing a series of washes with a suitable buffer between the addition of the sample (mites) and that of the detector reagent, some of the allergens extracted from the sample could be lost during the washings themselves, rendering the addition of the detector reagent ineffective. The aqueous solution in which the antibodies are dispersed would at the same time act to extract allergenic proteins from any mites present, enabling the analysis also to be carried out on dust samples and not necessarily on aqueous extracts previously prepared from the dust.

The method developed by the applicant could potentially be applied to the quantitative detection of various antigens or alternatively antibodies with particular specificity, thus making it extremely useful in all those situations, both in the human and in the veterinary field, where tests are required which are simple but sufficiently quick and sensitive for the diagnosis of particular pathological conditions. The examples which follow provide a detailed description of an embodiment of the testing method of the invention previously described.

### Example 1:

### - METHOD FOR THE DETECTION OF MITES

### 1a Preparation of the specific antibody

An anti-mite serum from rabbits obtained by immunisation by subcutaneous administrations at three-week intervals with a mite extract (protein content of 3 mg) dispersed in complete Freunds adjuvant (the first two immunisations) or incomplete Freunds adjuvant (the subsequent immunisations - at least four) is purified by affinity chromatography.

The fractions eluted with 0.1M glycine-HCl buffer, pH 2.7, are neutralised with 1 M phosphate buffer, pH 7.2 (PBS composition) combined in a pool and then the pool is dialysed against 10 mM sodium phosphate buffer and 2.7 mM NaCl, pH 7.4.

### 1bA Preparation of the dye

Several textile dyes are able to form coloured colloidal particles which are potentially interesting for diagnostic applications when combined with proteins (antibodies, antigens...). In particular, within the field of the invention it is preferred to use textile dyes of the types Brilliant Pink 5B, FBLN Blue, Red Samaron™ dyes (Hoechst). Alternatively the use of colloidal gold as a dye is envisaged in the field of the invention. In the case, for example, of the dye Brilliant Pink 5B™, a 5% solution of the dye in water is prepared giving a colloidal dispersion, this latter is then centrifuged 4 times at 20,000 g for 20 minutes and the sediment is then resuspended in a volume equal to the initial volume. Thereafter a final centrifuging is carried out at 125 g for 30 minutes in order to remove any very large aggregates. The supernatant liquor in the form of a colloidal dispersion is then analysed spectrophotometrically to determine the concentration of use.

For this purpose an aliquot is dissolved and diluted in ethanol to give an optical density of 1.000 as determined spectrophotometrically at the appropriate wavelength (540 for Brilliant Pink).

This value is indicated by the letter A.

The colloidal dispersion is stable for several months if kept at 4°C in the presence of thiomersal (0.01%).

### 1bB Preparation of the antibody-dye conjugate

Various concentrations of dye (from A=5 to A=50) were used to bind it to the antibodies (protein concentrations variable from 5 to 100 µg/ml) with the use of 10 mM phosphate buffer with a pH range variable from 6 to 8.5 and with NaCl concentrations of from 2.7 mM to 150 mM.

The experimental conditions which induced the greatest intensity of colour in the spot and hence gave the best signal were as follows:
phosphate buffer 10 mM, pH 7.4, NaCl 5 mM A=30 and an antibody concentration of 20µg/ml

In the preparation of the conjugate, suitable volumes of the dye and antibody to give a final concentration of the dye of A=30 and of the antibody of 20 µg/ml were placed in contact for 14 hours to enable the antibody to be adsorbed onto the coloured colloidal particles.

The conjugate was stabilised by the addition of a 1/5 volume of 5 mM NaCl, pH 7.4-30% BSA, incubation for one hour and then centrifuging at 12,000 g for 20 minutes at 4°C.

The sediment was taken up in a volume of buffer equal to the initial volume and constituted by 10 mM PBS, 5 mM NaCl, 5% BSA, 0.04% thiomersal, pH 7.4.

The conjugate thus prepared may be kept at 4°C (it is stable for short periods) or freeze-dried with an improvement in its stability up to at least six months.

### 1c Preparation of the reactive strip

Sticks (1 x 8 cm) constituted by a transparent substrate to which is fixed a small square (1 x 1 cm) of nitrocellulose paper having a porosity of 0.45 µm (bio-MAP, Agate Brianza, Milan), (preferred useful porosity range 0.2-5 µm) were immersed in water for 5 minutes and then dried. Subsequently 5 µg (in terms of protein content) of anti-mite antibodies purified by affinity chromatography were placed on each stick in a 10 µl volume of phosphate buffer (10 mM, 2.7 M NaCl) pH 7.4.

By this method the antibodies (proteins), in reacting with the nitrocellulose, form chemical bonds with the latter and remain fixed thereto.

The distribution of the solution containing the anti-mite antibodies may be carried out manually by means of a micropipette or automatically by a dispenser.

After the stick has been left to dry for 10 minutes in order to avoid aspecific reactions, the reactive sites on the nitrocellulose were blocked with 1.5% casein in PBS, pH 7.4 as the blocking protein.

After 2 hours of incubation at ambient temperature and under agitation, a series of washings was carried out with PBS-Tween 20; the reactive strip thus prepared was then dried or freeze-dried. The reactive strip thus remains stable for at least 6 months.

The reactive strip prepared as described above was placed in a suitable polyvinyl or polystyrene test tube; a quantity of house dust, generally from 60 to 80 mg, was then added to the latter by means of a measuring spoon. The house dust had previously been collected from the floor of the bedroom, from beds, from carpets or other household goods. Immediately thereafter the detector (1 - 1.5 ml), constituted by a solution containing the anti-acari antibodies purified by affinity chromatography and conjugated with the dye Brilliant Pink (as described previously), was added.

The presence of any mites was shown by the development of a coloured spot after a suitable incubation period (not less than 30 minutes). The coloured spot was made evident by washing of the reactive strip simply under flowing water. The addition to the test tube of house dust which was definitely negative (no mites as evaluated by microscopic observation) did not give rise to any coloured spot, showing the specificity of the method.

The sensitivity of the "one step" fixing method of the invention was shown by comparison with a "double step" testing method in which the steps of adding the sample under examination and the step of adding the detector were separated by intermediate washings both with the use of a mite extract and with the use of serum from patients known to be suffering from echinococcosis.

The comparative data are given in Table 1 from which the greater sensitivity of the "one step" method in the determination of mite allergens is seen.

A further confirmation of the effectiveness and sensitivity of the method was obtained by testing 5 samples of house dust by the method of the invention as described in Example 1 and by the quantitative laboratory method known as the ELISA test.

The results of the comparison are given in Table 2 from which it is seen that the quantities displayed correspond to a very good approximation to the data detectable by the quantitative method.

**TABLE 1**

| COMPARISON OF THE "ONE STEP" METHOD WITH THE "DOUBLE STEP" METHOD WITH THE USE OF AN EXTRACT OF MITES | | | |
|---|---|---|---|
| | | "one step" | "double step" |
| 1) | Mite extract from the strain D.p. (conc. µg/ml) | | |
| | 2 | ++++ | +++ |
| | 1 | ++++ | ++ |
| | 0.5 | ++++ | ++ |
| | 0.25 | +++ | + |

**TABLE 2**

| COMPARISON OF THE "ONE STEP" METHOD WITH THE ELISA TEST WITH SAMPLES OF HOUSE DUST | | |
|---|---|---|
| | "one step" | |
| "ELISA"* | | |
| | | |
| Sample 1 | + | < 0.4 µg/g |
| Sample 2 | ++++ | > 10 µg/g |
| Sample 3 | ++ | < 2 > 0.4 |
| µg/g | | |
| Sample 4 | +++ | < 10 > 2 |
| µg/g | | |
| Sample 5 | ++ | < 2 > 0.4 |
| µg/g | | |

| | | |
|---|---|---|
| * Quantity of Der p I | | |

## Claims

1. A sandwich type testing method for determining antigens mite in a test sample in which the test sample suspected of containing the antigen is placed in contact with a solid substrate having an antibody bound to its surface which can form an immunocomplex with the antigen and in which the formation of the complex is detected by the addition of a marked antibody **characterised in that** the solid substrate is placed in contact simultaneously with the test sample in the form of dust with the marked antibody.

2. A testing method according to Claim 1, in which the test sample and the marked antibody are premixed and the premixed product is placed in contact with the solid substrate.

3. A testing method according to Claim 1, **characterised in that** the marked antibody is an antibody conjugated with a colloidal dye or with colloidal gold.

4. A testing method according to claim 3, in which the colloidal dye is a textile dye.

5. A testing method according to claim 4, in which the textile dye is Brilliant Pink 5B.

6. A testing method according to claim 5, in which the solid substrate to which the antibody is bonded is a reactive strip whose reactive part has a porosity rage from 0,2 to 5 µm.

7. A testing method according to claim 6, in which the. porosity of the reactive part of the reactive strip is 0,45 µm.

8. A testing method according to claims 5 or 6, in which the reactive part of the reactive strip is constituted by nitrocellulose.

9. A testing method according to claims 1 to 8, in which the antibody bound to the solid substrate and the marked detector antibody are a polyclonal anti-mite antibody.

## Patentansprüche

1. Sandwich-Prüfverfahren zum Bestimmen von Milbenantigenen in einer Probe, bei dem die Probe, die verdächtigt wird, das Antigen zu enthalten, mit einem festen Substrat kontaktiert wird, an dessen Oberfläche ein Antikörper gebunden ist, der mit dem Antigen einen Immunkomplex bilden kann, und bei dem die Komplexbildung durch Zugabe eines markierten Antikörpers ermittelt wird, **dadurch gekennzeichnet, dass** das feste Substrat mit der Probe in Form von Staub und mit dem markierten Antikörper gleichzeitig kontaktiert wird.

2. Prüfverfahren nach Anspruch 1, bei dem die Probe und der markierte Antikörper vorgemischt sind und das Vormischprodukt mit dem festen Substrat kontaktiert wird.

3. Prüfverfahren nach Anspruch 1, **dadurch gekennzeichnet, dass** der markierte Antikörper ein mit einem kolloidalen Farbstoff oder mit kolloidalem Gold konjugierter Antikörper ist.

4. Prüfverfahren nach Anspruch 3, bei dem der kolloidale Farbstoff ein Textilfarbstoff ist.

5. Prüfverfahren nach Anspruch 4, bei dem der Textilfarbstoff Brilliant Pink 5B ist.

6. Prüfverfahren nach Anspruch 5, bei dem das feste Substrat, an den der Antikörper gebunden ist, ein reaktionsfähiger Streifen ist, dessen reaktionsfähiger Teil eine Porosität im Bereich von 0,2 bis 5 µm hat.

7. Prüfverfahren nach Anspruch 6, bei dem die Porosität des reaktionsfähigen Teils des reaktionsfähigen Streifens 0,45 µm beträgt.

8. Prüfverfahren nach Anspruch 5 oder 6, bei dem der reaktionsfähige Teil des reaktionsfähigen Streifens aus Nitrocellulose besteht.

9. Prüfverfahren nach Anspruch 1 bis 8, bei dem der an das feste Substrat gebundene Antikörper und der markierte Antikörper ein polyclonaler Antimilben-Antikörper sind.

## Revendications

1. Méthode d'essai de type sandwich pour doser des antigènes d'acariens dans un prélèvement, dans laquelle on place le prélèvement, suspecté contenir l'antigène, au contact d'un substrat solide possédant un anticorps lié à sa surface qui peut former un immunocomplexe avec l'antigène, et dans laquelle la formation du complexe est détectée par addition d'un anticorps marqué, **caractérisée en ce que** le substrat solide est placé simultanément au contact du prélèvement sous forme de poussière et de l'anticorps marqué.

2. Méthode d'essai selon la revendication 1, dans laquelle le prélèvement et l'anticorps marqué sont mélangés au préalable et le produit mélangé au préalable est placé au contact du substrat solide.

3. Méthode d'essai selon la revendication 1, **caractérisée en ce que** l'anticorps marqué est un anticorps conjugué avec un colorant colloïdal ou avec de l'or colloïdal.

4. Méthode d'essai selon la revendication 3, dans laquelle le colorant colloïdal est un colorant textile.

5. Méthode d'essai selon la revendication 4, dans laquelle le colorant textile est le Brilliant Pink 5B.

6. Méthode d'essai selon la revendication 5, dans laquelle le substrat solide auquel est lié l'anticorps est une bande réactive dont la partie réactive présente une plage de porosité de 0,2 à 5 µm.

7. Méthode d'essai selon la revendication 6, dans laquelle la porosité de la partie réactive de la bande réactive est de 0,45 µm.

8. Méthode d'essai selon les revendications 5 ou 6, dans laquelle la partie réactive de la bande réactive est constituée de nitrocellulose.

9. Méthode d'essai selon les revendications 1 à 8, dans laquelle l'anticorps lié au substrat solide et l'anticorps détecteur marqué sont un anticorps polyclonal anti-acariens.
